# EUROPÄISCHE PATENTSCHRIFT

(11) **EP 1 689 745 B1**
(45) Veröffentlichungstag und Bekanntmachung des Hinweises auf die Patenterteilung: **15.10.2008**
(21) Anmeldenummer: 04787027.4
(22) Anmeldetag: 27.09.2004
(51) Int. Cl.: C07D 417/06, A61K 31/425, A61P 35/00

(54) **NEUE MAKROCYCLEN ZUR BEHANDLUNG VON KREBSERKRANKUNGEN**
NOVEL MACROCYCLES FOR TREATING TUMOR DISEASES
NOUVEAUX MACROCYCLES POUR TRAITER DES MALADIES CANCEREUSES

(30) Priorität: 26.09.2003 DE 10344882
(43) Veröffentlichungstag der Anmeldung: 16.08.2006
(73) Patentinhaber: Richter, Wolfgang, 81243 München (DE)
(72) Erfinder: RICHTER, Wolfgang, 81243 München (DE); HENKEL, Bernd, 82152 Planegg (DE); CAPPI, Michael, 81369 München (DE)
(74) Vertreter: Maiwald Patentanwalts GmbH
(86) Internationale Anmeldenummer: PCT/EP2004/010820
(87) Internationale Veröffentlichungsnummer: WO 2005/030767

(56) Entgegenhaltungen:
- WO-A-93/10121
- WO-A-99/02514

## Beschreibung

Epothilone (DE 4138042) sind Naturstoffe mit außerordentlicher biologischer Wirkung, z.B. als Mitosehemmer, Mikrotubuli-modifizierende Agenzien, Cytotoxica oder Fungizide. Insbesondere verfügen sie über Paclitaxel-ähnliche Eigenschaften und übertreffen Paclitaxel (Taxol^{®}) in einigen Tests noch an Aktivität. Einige Derivate befinden sich derzeit in klinischen Studien zur Behandlung von Krebsleiden (Nicolaou et al. Angew. Chem. Int. Ed. 1998, 37, 2014-2045; Flörsheimer et al. Expert Opin. Ther. Patents 2001, 11, 951-968).

Ziel der vorliegenden Erfindung war es, neue epothilonartige Derivate bereitzustellen, die ein besseres Profil bezüglich ihres präklinischen und klinischen Entwicklungspotentials aufweisen.

Die vorliegende Erfindung betrifft Verbindungen der allgemeinen Formel (I): 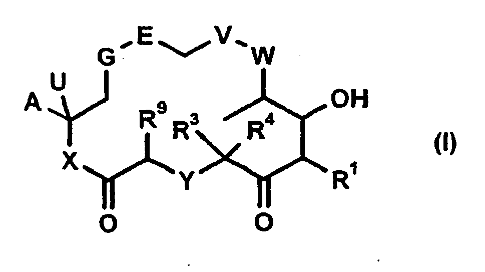 worin
A ein Heteroalkyl-, ein Heterocycloalkyl-, ein Heteroalkylcycloalkyl-, ein Heteroaryl- oder ein Heteroarylalkylrest ist,
U ein Wasserstoffatom, ein Heteroalkyl-, ein Heterocycloalkyl-, ein Heteroalkylcycloalkyl-, ein Heteroaryl- oder ein Heteroarylalkylrest ist,
G-E aus folgenden Gruppen ausgewählt ist, 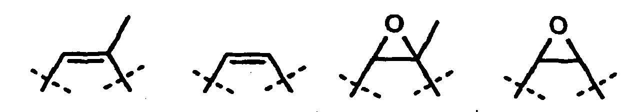
oder Teil eines gegebenenfalls substituierten Phenylrings ist,
V-W eine Gruppe der Formel CH-CH oder C=C (cis oder trans) ist,
R¹ eine C₁-C₄-Alkyl- oder eine C₃-C₄-Cycloalkylgruppe ist,
X ein Sauerstoffatom oder eine Gruppe der Formel NR² ist, wobei R² ein Wasserstoffatom, ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest ist,
Y ein Sauerstoffatom oder eine Gruppe der Formel NR¹⁰ ist, wobei R¹⁰ ein Wasserstoffatom, ein Sauerstoffatom (N-Oxid), eine OH, NH₂, Alkyl- oder eine Heteroalkylgruppe (wie z. B. eine Alkyloxy-, Alkylamino- oder Dialkylaminogruppe) ist.
R³ und R⁴ unabhängig voneinander ein Wasserstoffatom, eine C₁-C₄-Alkylgruppe oder zusammen Teil einer Cycloalkylgruppe mit 3 oder 4 Ringatomen sind,
R⁹ ein Wasserstoffatom, ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest ist und
oder ein pharmakologisch akzeptables Salz, Solvat, Hydrat oder eine pharmakologisch akzeptable Formulierung derselben.

Der Ausdruck Alkyl bezieht sich auf eine gesättigte, geradkettige oder verzweigte Kohlenwasserstoffgruppe, die 1 bis 20 Kohlenstoffatome, vorzugsweise 1 bis 12 Kohlenstoffatome, besonders bevorzugt 1 , bis 6 Kohlenstoffatome aufweist, z.B. die Methyl-, Ethyl-, Isopropyl-, Isobutyl-, tert-Butyl, n-Hexyl-, 2,2-Dimethylbutyl- oder n-Octylgruppe.

Die Ausdrücke Alkenyl und Alkinyl beziehen sich auf zumindest teilweise ungesättigte, geradkettige oder verzweigte Kohlenwasserstoffgruppen, die 2 bis 20 Kohlenstoffatome, vorzugsweise 2 bis 12 Kohlenstoffatome, besonders bevorzugt 2 bis 6 Kohlenstoffatome aufweisen, z. B. die Allyl-, Acetylenyl-, Propargyl-, Isoprenyl- oder Hex-2-enyl-Gruppe.

Der Ausdruck.Heteroalkyl bezieht sich auf eine Alkyl-, eine Alkenyl- oder eine Alkinyl-Gruppe, in der ein oder mehrere (bevorzugt 1, 2 oder 3) Kohlenstoffatome durch ein Sauerstoff-, Stickstoff-, Phosphor-, Bor- oder Schwefelatom ersetzt sind (bevorzugt Sauerstoff, Schwefel oder Stickstoff), z.B. eine Alkyloxy-Gruppe wie z.B. Methoxy oder Ethoxy, oder eine Methoxymethyl-, Nitril-, Methyl-carboxyalkylester- oder 2,3-Dioxyethyl-Gruppe. Der Ausdruck Heteroalkyl bezieht sich des weiteren auf eine Carbonsäure oder eine von einer Carbonsäure abgeleitete Gruppe wie z. B. Acyl, Acyloxy, Carboxyalkyl, Carboxyalkylester z.B. Methyl-carboxyalkylester, Carboxyalkylamid, Alkoxycarbonyl oder Alkoxycarbonyloxy.

Der Ausdruck Cycloalkyl bzw. Cyclo- bezieht sich auf eine gesättigte oder teilweise ungesättigte cyclische Gruppe, die einen oder mehrere Ringe aufweist, die ein Gerüst bilden, welches 3 bis 14 Kohlenstoffatome, vorzugsweise 3 bis 10 Kohlenstoffatome enthält, z.B. die Cyclopropyl-, Cyclohexyl-, Tetralin- oder Cyclohex-2-enyl-Gruppe.

Der Ausdruck Heterocycloalkyl bzw. Heterocyclo- bezieht sich auf eine Cycloalkylgruppe wie oben definiert, in der ein oder mehrere (bevorzugt 1, 2 oder 3) Kohlenstoffatome durch ein Sauerstoff-, Stickstoff-, Phosphor- oder Schwefelatom ersetzt sind und kann beispielsweise für die Piperidin-, Morpholin-, Tetrahydrofuran-, Tetrahydrothiophen-, N-Methylpiperazin- oder N-Phenylpiperazingruppe stehen.

Die Ausdrücke Alkylcycloalkyl bzw. Heteroalkylcycloalkyl beziehen sich auf Gruppen, die entsprechend den obigen Definitonen sowohl Cycloalkyl- bzw. Heterocycloalkyl- wie auch Alkyl-, Alkenyl-, Alkinyl- und/oder Heteroalkylgruppen enthalten.

Der Ausdruck Aryl bzw. Ar bezieht sich auf eine aromatische Gruppe, die einen oder mehrere Ringe hat, und durch ein Gerüst gebildet wird, das 5 bis 14 Kohlenstoffatome, vorzugsweise 5 oder 6 bis 10 Kohlenstoffatome enthält z.B. eine Phenyl-, Naphthyl-, 2-, 3- oder 4-Methoxyphenyl-, 2-, 3- oder 4-Ethoxyphenyl-, 4-Carboxyphenylalkyl- oder 4-Hydroxyphenyl-Gruppe.

Der Ausdruck Heteroaryl bezieht sich auf eine ArylGruppe, in der ein oder mehrere (bevorzugt 1, 2 oder 3) Kohlenstoffatome durch ein Sauerstoff-, Stickstoff-, Phosphor- oder Schwefelatom ersetzt sind, z.B. die 4-Pyridyl-, 2-Imidazolyl-, 3-Pyrazolyl-, Oxazolyl-, Thiazolyl-, Thiophen- und Isochinolinyl-Gruppe.

Die Ausdrücke Aralkyl bzw. Heteroaralkyl beziehen sich auf Gruppen, die entsprechend den obigen Definitionen sowohl Aryl- bzw. Heteroaryl- wie auch Alkyl-, Alkenyl-, Alkinyl- und/oder Heteroalkyl- und/oder Cycloalkyl- und/oder Heterocycloalkylgruppen enthalten, z.B. die Tetrahydroisochinolinyl-, Benzyl-, 2- oder 3-Ethylindolyl- oder 4-Methylpyridino-Gruppe.

Die Ausdrücke Alkyl, Alkenyl, Alkinyl, Heteroalkyl, Cycloalkyl, Heterocycloalkyl, Aryl, Heteroaryl, Aralkyl und Heteroaralkyl sowie "gegebenenfalls substituiert" beziehen sich auch auf Gruppen, in denen ein oder mehrere Wasserstoffatome solcher Gruppen durch Fluor-, Chlor-, Brom- oder Jodatome oder OH, SH, NH₂ oder NO₂-Gruppen ersetzt sind. Diese Ausdrücke beziehen sich weiterhin auf Gruppen, die mit unsubstituierten Alkyl-, Alkenyl-, Alkinyl-, Heteroalkyl-, Cycloalkyl-, Heterocycloalkyl-, Aryl-, Heteroaryl-, Aralkyl- oder Heteroaralkyl-Gruppen substituiert sind.

Verbindungen der Formel (I) können aufgrund ihrer Substitution ein oder mehrere Chiralitätszentren enthalten. Die vorliegende Erfindung umfasst daher sowohl alle reinen Enantiomere und alle reinen Diastereomere, als auch deren Gemische in jedem Mischungsverhältnis. Des weiteren sind von der vorliegenden Erfindung auch alle cis/trans-Isomeren der Verbindungen der allgemeinen Formel (I) sowie Gemische davon umfasst.

Bevorzugt sind Verbindungen der Formel (I) wobei A eine Gruppe der Formel -C(CH₃)=CHR⁵ oder -CH=CHR⁵ ist, wobei R⁵ ein Heteroaryl- oder ein Heteroarylalkylrest ist.

Des weiteren bevorzugt sind Verbindungen der Formel (I) wobei A die allgemeine Formel (II) oder (III) aufweist: 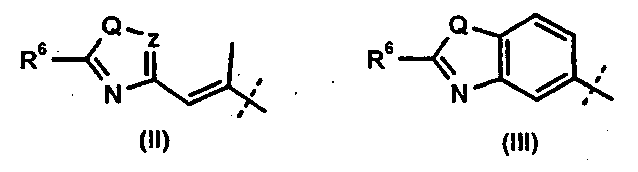 wobei Q ein Schwefelatom, ein Sauerstoffatom oder eine Gruppe der Formel NR⁷ ist, wobei R⁷ ein Wasserstoffatom, eine C₁-C₄-Alkylgruppe oder eine C₁-C₄-Heteroalkylgruppe ist, z ein Stickstoffatom oder eine CH-Gruppe ist und R⁶ eine Gruppe der Formel OR⁸ oder NHR⁸, eine Alkyl-, Alkenyl, Alkinyl- oder eine Heteroalkylgruppe (bevorzugt eine Gruppe der Formel CH₂OR⁸ oder CH₂NHR⁸) ist, wobei R⁸ ein Wasserstoffatom, eine C₁-C₄-Alkylgruppe oder eine C₁-C₄-Heteroalkylgruppe (bevorzugt ein Wasserstoffatom) ist.

Besonders bevorzugt ist z eine CH-Gruppe.

Wiederum bevorzugt sind Verbindungen der Formel (I) wobei Q ein Schwefelatom oder ein Sauerstoffatom ist.

Besonders bevorzugt sind Verbindungen der Formel (I) wobei R⁶ eine Gruppe der Formel CH₃, CH₂OH oder CH₂NH₂ ist.

Weiter bevorzugt ist U ein Wasserstoffatom, eine CF₃ oder eine Methylgruppe (besonders bevorzugt ein Wasserstoffatom).

Weiter bevorzugt ist R² ein Wasserstoffatom oder eine C₁-C₄-Alkylgruppe (besonders bevorzugt ein Wasserstoffatom).

Des weiteren bevorzugt sind Verbindungen der Formel (I) wobei X ein Sauerstoffatom ist.

Ausserdem ist R¹ bevorzugt eine Methyl-, Ethyl- oder eine Propylgruppe; besonders bevorzugt eine Methylgruppe.

Wiederum bevorzugt sind R³ und R⁴ Methylgruppen.

Weiter bevorzugt ist R⁹ die Seitenkette einer natürlichen Aminosäure; insbesondere ein Wasserstoffatom.

Des weiteren bevorzugt ist Y ein Sauerstoffatom oder eine Gruppe der Formel NH, NOH oder NO.

Beispiele für pharmakologisch akzeptable Salze der Verbindungen der Formel (I) sind Salze (oder Mischsalze) von physiologisch akzeptablen Mineralsäuren wie Salzsäure, Schwefelsäure und Phosphorsäure oder Salze von organischen Säuren wie Methansulfonsäure, p-Toluolsulfonsäure, Milchsäure, Essigsäure, Trifluoressigsäure, Zitronensäure, Bernsteinsäure, Fumarsäure, Maleinsäure und Salicylsäure. Verbindungen der Formel (I) können solvatisiert, insbesondere hydratisiert sein. Die Hydratisierung kann z.B. während des Herstellungsverfahrens oder als Folge der hygroskopischen Natur der anfänglich wasserfreien Verbindungen der Formel (I) auftreten. Wenn die Verbindungen der Formel (I) asymmetrische C-Atome enthalten, können sie entweder als achirale Verbindungen, Diastereomeren-Gemische, Gemische von Enantiomeren oder als optisch reine Verbindungen vorliegen. Des weiteren sind von der vorliegenden Erfindung auch alle cis/trans-Isomeren der vorliegenden Verbindungen der allgemeinen Formel (I) sowie Gemische davon umfasst.

Die pharmazeutischen Zusammensetzungen gemäß der vorliegenden Erfindung enthalten mindestens eine Verbindung der Formel (I) als Wirkstoff und fakultativ Trägerstoffe und/oder Adjuvantien.

Die Pro-Drugs (siehe z. B. R. B. Silverman, Medizinische Chemie, VCH Weinheim, 1995, Kapitel 8, S. 361ff), bestehen aus einer Verbindung der Formel (I) und mindestens einer pharmakologisch akzeptablen Schutzgruppe, die unter physiologischen Bedingungen abgespalten wird, z.B. einer Alkoxy-, Aralkyloxy-, Acyl- oder Acyloxy-Gruppe, wie z.B. einer Ethoxy-, Benzyloxy-, Acetyl- oder Acetyloxy-Gruppe.

Ausser den bereits beschriebenen Krebserkrankungen sind die Verbindungen der vorliegenden Erfindung bei der Behandlung von weiteren Erkrankungen wie Autoimmunkrankheiten, entzündlichen Krankheiten, Tumorerkrankungen und sonstigen Erkrankungen, die auf eine Störung des Zellwachstums zurückzuführen sind von grossem Interesse.

Verbindungen der Formel (I), ihrer pharmakologisch akzeptablen Salze bzw. Solvate und Hydrate sowie Formulierungen und pharmazeutischen Zusanunensetzungen zur therapeutischen Verwendung liegen enbenfalls im Rahmen der vorliegenden Erfindung.

Auch die Verwendung dieser Wirkstoffe zur Herstellung von Arzneimitteln zur Behandlung von Krebserkrankungen ist Gegenstand der vorliegenden Erfindung. Im allgemeinen werden Verbindungen der Formel (I) unter Anwendung der bekannten und akzeptablen Modi, entweder einzeln oder in Kombination mit einem beliebigen anderen therapeutischen Mittel verabreicht. Solche therapeutisch nützlichen Mittel können auf einem der folgenden Wege verabreicht werden: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, transdermal oder intranasal. Zur Herstellung solcher Tabletten, Pillen, Halbfeststoffe, überzogenen Tabletten, Dragees und harten Gelatinekapseln kann das therapeutisch verwendbare Produkt mit pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch und dgl. Zur Herstellung von weichen Kapseln kann man Arzneimittelträgerstoffe wie z.B. pflanzliche Öle, Petroleum, tierische oder synthetische Öle, Wachs, Fett, Polyole einsetzen. Zur Herstellung von flüssigen Lösungen und Sirups kann man Arzneimittelträgerstoffe wie z.B. Wasser, Alkohole, wäßrige Salzlösung, wäßrige Dextrose, Polyole, Glycerin, pflanzliche Öle, Petroleum, tierische oder synthetische Öle verwenden. Für Suppositorien kann man Arzneimittelträgerstoffe wie z.B. pflanzliche Öle, Petroleum, tierische oder synthetische Öle, Wachs, Fett und Polyole verwenden. Für Aerosol-Formulierungen kann man komprimierte Gase, die für diesen Zweck geeignet sind, wie z.B. Sauerstoff, Stickstoff, Edelgase und Kohlendioxid einsetzen. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Antioxidantien enthalten.

Kombinationen mit anderen therapeutischen Mitteln können weitere Wirkstoffe beinhalten, die gewöhnlich zur Behandlung von Krebserkrankungen eingesetzt werden.

Zur Behandlung von Krebserkrankungen kann die Dosis der erfindungsgemäßen biologisch aktiven Verbindung innerhalb breiter Grenzen variieren und kann auf den individuellen Bedarf eingestellt werden. Im allgemeinen ist eine Dosis von 1 µg bis 100 mg/kg Körpergewicht pro Tag geeignet, wobei eine bevorzugte Dosis 10 µg bis 25 mg/kg pro Tag ist. In geeigneten Fällen kann die Dosis auch unter oder über den oben angegebenen Werten liegen.

### Beispiele

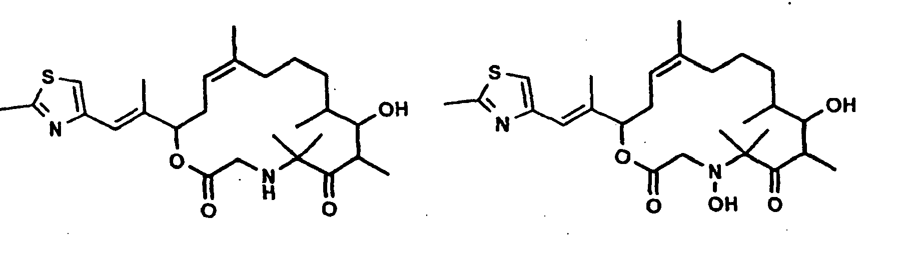

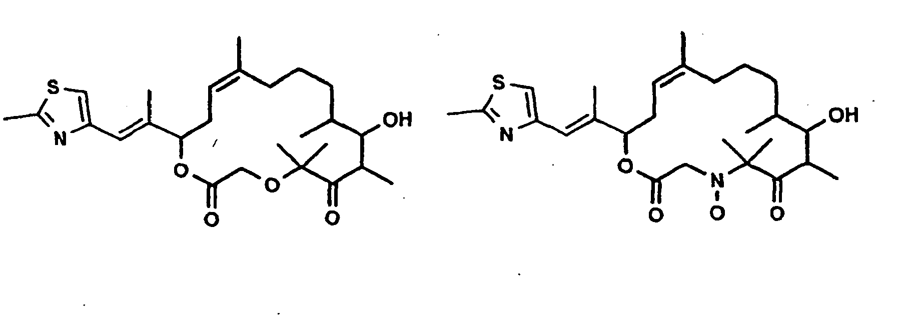

## Patentansprüche

1. Verbindungen der allgemeinen Formel (I) 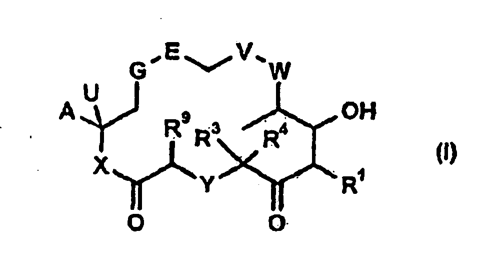 worin
A ein Heteroalkyl-, ein Heterocycloalkyl-, ein Heteroalkylcycloalkyl-, ein Heteroaryl- oder ein Heteroarylalkylrest ist,
U ein Wasserstoffatom, ein Heteroalkyl-, ein Heterocycloalkyl-, ein Heteroalkylcycloalkyl-, ein Heteroaryl- oder ein Heteroarylalkylrest ist,
G-E aus folgenden Gruppen ausgewählt ist, 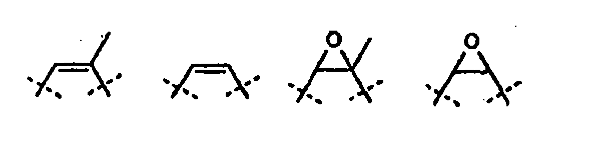
oder Teil eines gegebenenfalls substituierten Phenylrings ist,
V-W eine Gruppe der Formel CH-CH oder C=C (cis oder trans) ist,
R¹ eine C₁-C₄-Alkyl-, oder eine C₃-C₄-Cycloalkylgruppe ist,
X ein Sauerstoffatom oder eine Gruppe der Formel NR² ist, wobei R² ein Wasserstoffatom, ein Alkyl-, Alkenyl-, Alkinyl-, Heteroalkyl-, Aryl-, Heteroaryl-, Cycloalkyl-,
Alkylcycloalkyl-, Heteroalkylcycloalkyl-, Heterocycloalkyl-, Aralkyl- oder ein Heteroaralkylrest ist,
Y ein Sauerstoffatom oder eine Gruppe der Formel NR¹⁰ ist, wobei R¹⁰ ein Wasserstoffatom, ein Sauerstoffatom, eine OH, NH₂, Alkyl- oder eine Heteroalkylgruppe ist,
R³ und R⁴ unabhängig voneinander ein Wasserstoffatom, eine C₁-C₄-Alkylgruppe oder zusammen Teil einer Cycloalkylgruppe mit 3 oder 4 Ringatomen sind,
R⁹ ein Wasserstoffatom, ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest ist,
oder ein pharmakologisch akzeptables Salz, Solvat oder Hydrat.

2. Verbindungen nach Anspruch 1, wobei A eine Gruppe der Formel -C(CH₃)=CHR⁵ oder -CH=CHR⁵ ist, wobei R⁵ ein Heteroaryl- oder ein Heteroarylalkylrest ist.

3. Verbindungen nach Anspruch 1, wobei A die allgemeine Formel (II) oder (III) aufweist: 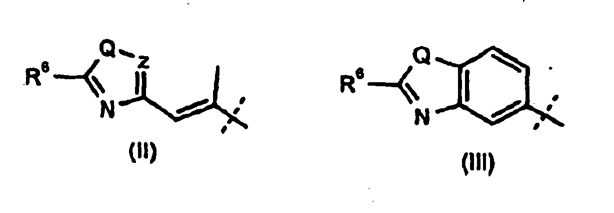 worin
Q ein Schwefelatom, ein Sauerstoffatom oder eine Gruppe der Formel NR⁷ ist, wobei R⁷ ein Wasserstoffatom, eine C₁-C₄-Alkylgruppe oder eine C₁-C₄-Heteroalkylgruppe ist, z ein Stickstoffatom oder eine CH-Gruppe ist und R⁶ eine Gruppe der Formel OR⁸ oder NHR⁸, eine Alkyl-, Alkenyl-, Alkinyl- oder eine Heteroalkylgruppe ist, wobei R⁸ ein Wasserstoffatom, eine C₁-C₄-Alkylgruppe oder eine C₁-C₄-Heteroalkylgruppe ist.

4. Verbindungen nach Anspruch 3, wobei z eine CH-Gruppe ist.

5. Verbindungen nach Anspruch 3 oder 4, wobei Q ein Schwefelatom oder ein Sauerstoffatom ist.

6. Verbindungen nach einem der Ansprüche 3 bis 5, wobei R⁶ eine Gruppe der Formel CH₃, CH₂OH oder CH₂NH₂ ist.

7. Verbindungen nach einem der Ansprüche 1 bis 6, wobei X ein Sauerstoffatom ist.

8. Verbindungen nach einem der Ansprüche 1 bis 7, wobei R¹ eine Methylgruppe ist.

9. Verbindungen nach einem der Ansprüche 1 bis 8, wobei R³ und R⁴ Methylgruppen sind.

10. Verbindungen nach einem der Ansprüche 1 bis 9, wobei U ein Wasserstoffatom ist.

11. Verbindungen nach einem der Ansprüche 1 bis 10, wobei R⁹ ein Wasserstoffatom ist.

12. Verbindungen nach einem der Ansprüche 1 bis 11, wobei Y ein Sauerstoffatom oder eine Gruppe der Formel NH, NOH oder NO ist.

13. Pharmazeutische Zusammensetzung, die eine Verbindung nach einem der Ansprüche 1 bis 12 und fakultativ Trägerstoffe und/oder Adjuvanzien enthalten.

14. Verbindung oder pharmazeutische Zusammensetzung nach einem der Ansprüche 1 bis 13 zur Behandlung von Krebserkrankungen.

## Claims

1. Compound of the general formula (I), 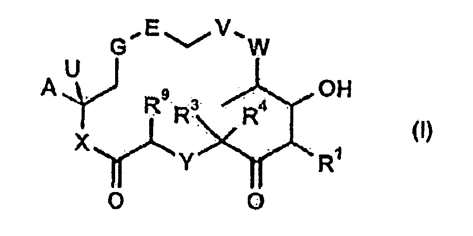 wherein
A is a heteroalkyl-, heterocycloalkyl-, heteroalkylcycloalkyl-, heteroaryl- or heteroarylalkyl group;
U is hydrogen, a heteroalkyl-, heterocycloalkyl-, heteroalkylcycloalkyl-, heteroaryl- or heteroarylalkyl group;
G-E is selected from the following groups 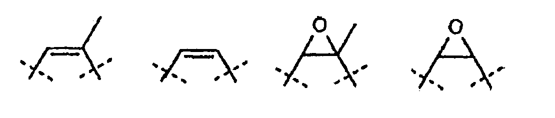
or is part of an optionally substituted phenyl ring;
V-W is a group of the formula CH-CH or C=C (cis or trans);
R¹ is a C₁-C₄ alkyl- or C₃-C₄ cycloalkyl group;
X is oxygen or a group of the formula NR², wherein R² is hydrogen, an alkyl-, alkenyl-, alkinyl-, heteroalkyl-, aryl-, heteroaryl-, cycloalkyl-, alkylcycloalkyl-, heteroalkylcycloalkyl-, heterocycloalkyl-, aralkyl- or heteroaralkyl group;
Y is oxygen or a group of the formula R¹⁰, wherein R¹⁰ is hydrogen, oxygen, a OH-, NH₂-, alkyl- or heteroalkyl group;
R³ and R⁴ are independently of each other hydrogen, a C₁-C₄ alkyl group or together are part of a cycloalkyl group with 3 or 4 ring atoms;
R⁹ is hydrogen, an alkyl-, alkenyl-, alkinyl-, heteroalkyl-, aryl-, heteroaryl-, cycloalkyl-, alkylcycloalkyl-, heteroalkylcycloalkyl-, heterocycloalkyl-, aralkyl- or heteroaralkyl group;
or a pharmacologically acceptable salt, solvate or hydrate thereof.

2. The compound of claim 1, wherein A is a group of the formula -C(CH₃)=CHR⁵ or -CH=CHR⁵, wherein R⁵ is a heteroaryl- or heteroarylalkyl group.

3. The compound of claim 1, wherein A has the general formula (II) or (III), 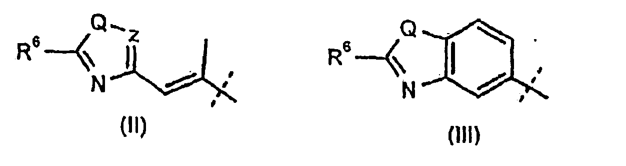 wherein
Q is sulfur, oxygen or a group of the formula NR⁷, wherein R⁷ is hydrogen, a C₁-C₄ alkyl group or a C₁-C₄ heteroalkyl group; z is nitrogen or a CH group; and R⁶ is a group of the formula OR⁸ or NHR⁸, an alkyl-, alkenyl-, alkinyl- or heteroalkyl group, wherein R⁸ is hydrogen, a C₁-C₄ alkyl group or a C₁-C₄ heteroalkyl group.

4. The compound of claim 3, wherein z is a CH group.

5. The compound of claim 3 or 4, wherein Q is sulfur or oxygen.

6. The compound of any of claims 3 to 5, wherein R⁶ is a group of the formula CH₃, CH₂OH or CH₂NH₂.

7. The compound of any of claims 1 to 6, wherein X is oxygen.

8. The compound of any of claims 1 to 7, wherein R¹ is a methyl group.

9. The compound of any of claims 1 to 8, wherein R³ and R⁴ are methyl groups.

10. The compound of any of claims 1 to 9, wherein U is hydrogen.

11. The compound of any of claims 1 to 10, wherein R⁹ is hydrogen.

12. The compound of any of claims 1 to 11, wherein Y is oxygen or a group of the formula NH, NOH or NO.

13. Pharmaceutical composition comprising a compound of any of claims 1 to 12 and optionally carriers and/or adjuvants.

14. The compound or the pharmaceutical composition of any of claims 1 to 13 for the treatment of cancer.

## Revendications

1. Composés de formule générale (I) 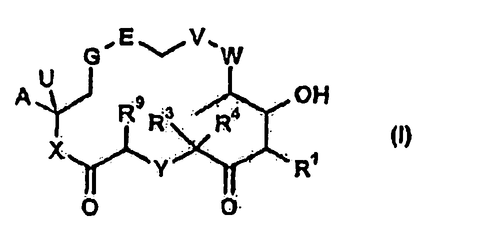 dans lesquels
A est un résidu hétéroalkyle, hétérocycloalkyle, hétéroalkylcycloalkyle, hétéroaryle ou hétéroarylalkyle,
U est un atome d'hydrogène, un résidu hétéroalkyle, hétérocycloalkyle, hétéroalkylcycloalkyle, hétéroaryle ou hétéroarylalkyle,
G-E est choisi parmi les groupes suivants, 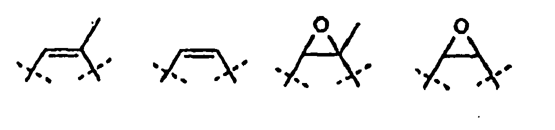 ou est le cas échéant une partie d'un anneau phényle substitué,
V-W est un groupe de formule CH-CH ou C=C (cis ou trans),
R¹ est un groupe C₁-C₄-alkyle ou C₃-C₄-cycloalkyle,
X est un atome d'oxygène ou un groupe de formule NR², dans lequel R² est un atome d'hydrogène, un résidu alkyle, alkényle, alkinyle, hétéroalkyle, aryle, hétéroaryle, cycloalkyle, alkylcycloalkyle, hétéroalkylcycloalkyle, hétérocycloalkyle, aralkyle ou hétéroaralkyle,
Y est un atome d'oxygène ou un groupe de formule NR¹⁰ dans lequel R¹⁰ est un atome d'hydrogène, un atome d'oxygène, un groupe OH, NH₂, alkyle ou hétéroalkyle,
R³ et R⁴ sont indépendamment l'un de l'autre un atome d'hydrogène, un groupe C₁-C₄-alkyle ou font ensemble partie d'un groupe cycloalkyle avec 3 ou 4 anneaux atomiques,
R⁹ est un atome d'hydrogène, un résidu alkyle, alkényle, alkinyle, hétéroalkyle, aryle, hétéroaryle, cycloalkyle, alkylcycloalkyle, hétéroalkylcycloalkyle, hétérocycloalkyle, aralkyle ou hétéroaralkyle,
ou un sel, un solvate ou un hydrate pharmacologiquement acceptable.

2. Composés selon la revendication 1, dans lesquels A est un groupe de formule -C(CH₃)=CHR⁵ ou -CH=CHR⁵, dans lequel R⁵ est un résidu hétéroaryle ou hétéroarylalkyle.

3. Composés selon la revendication 1, dans lesquels A comporte la formule générale (II)
ou (III) : 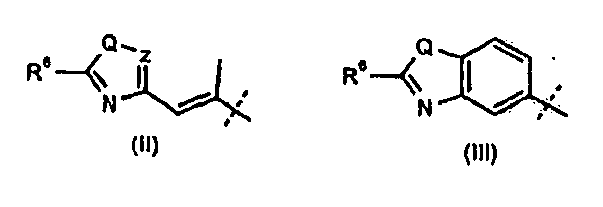 dans lesquels
Q est un atome de soufre, un atome d'oxygène ou un groupe de formule NR⁷ dans lequel R⁷ est un atome d'hydrogène, un groupe C₁-C₄-alkyle ou un groupe C₁-C₄-hétéroalkyle, z est un atome d'azote ou un groupe CH et R⁶ est un groupe de formule OR⁸ ou NHR⁸, un groupe alkyle, alkényle, alkinyle, ou hétéroalkyle, dans lequel R⁸ est un atome d'hydrogène, un groupe C₁-C₄-alkyle ou un groupe C₁-C₄-hétéroalkyle.

4. Composés selon la revendication 3, dans lesquels z est un groupe CH.

5. Composés selon la revendication 3 ou 4, dans lesquels Q est un atome de soufre ou un atome d'oxygène.

6. Composés selon l'une des revendications 3 à 5, dans lesquels R⁶ est un groupe de formule CH₃, CH₂OH ou CH₂NH₂.

7. Composés selon l'une des revendications 1 à 6, dans lesquels X est un atome d'oxygène.

8. Composés selon l'une des revendications 1 à 7, dans lesquels R¹ est un groupe méthyle.

9. Composés selon l'une des revendications 1 à 8, dans lesquels R³ et R⁴ sont des groupes méthyles.

10. Composés selon l'une des revendications 1 à 9, dans lesquels U est un atome d'hydrogène.

11. Composés selon l'une des revendications 1 à 10, dans lesquels R⁹ est un atome d'hydrogène.

12. Composés selon l'une des revendications 1 à 11, dans lesquels Y est un atome d'oxygène ou un groupe de formule NH, NOH ou NO.

13. Composition pharmaceutique qui contient un composé selon l'une des revendications 1 à 12 et facultativement des supports et/ou des adjuvants.

14. Composé ou composition pharmaceutique selon l'une des revendications 1 à 13 pour le traitement de maladies cancereuses.
